# EUROPÄISCHE PATENTANMELDUNG

(11) **EP 3 725 300 A1**
(43) Veröffentlichungstag der Anmeldung: **21.10.2020**
(21) Anmeldenummer: 20168706.8
(22) Anmeldetag: 08.04.2020
(51) Int. Cl.: A61K 9/00, A61K 9/70, A61K 31/4196, A61K 47/02, A61K 47/10, A61K 47/14

(54) **TRANSDERMALES DARREICHUNGSSYSTEM MIT AROMATASEHEMMER**

(30) Priorität: 18.04.2019 DE 102019110398
(71) Anmelder: AMW GmbH, 83627 Warngau (DE)
(72) Erfinder: Sonntag, Verena, 85640 Putzbrunn (DE); Gasteiger, Lisa-Marie, 83735 Bayrischzell (DE); Ritter, Elisabeth, 83627 Warngau (DE); Huber, Ludwig, 83626 Valley (DE)
(74) Vertreter: Beckord & Niedlich Patentanwälte PartG mbB

(57) **Zusammenfassung**

Die vorliegende Erfindung betrifft ein transdermales Darreichungssystem (TDS) umfassend zumindest einen Aromatasehemmer, wobei der zumindest eine Aromatasehemmer in einer Matrix vorliegt, welche zumindest ein Polymer aus der Klasse der Silicone enthält. Des Weiteren betrifft die vorliegende Erfindung ein Verfahren zur Herstellung des erfindungsgemäßen TDS sowie die Verwendung des erfindungsgemäßen TDS.

## Beschreibung

Die vorliegende Erfindung betrifft ein transdermales Darreichungssystem (TDS) umfassend zumindest einen Aromatasehemmer, wobei der zumindest eine Aromatasehemmer in einer Matrix vorliegt, welche zumindest ein Polymer aus der Klasse der Silicone enthält. Des Weiteren betrifft die vorliegende Erfindung ein Verfahren zur Herstellung des erfindungsgemäßen TDS sowie die Verwendung des erfindungsgemäßen TDS.

Die transdermale Verabreichung von Arzneimittel-Wirkstoffen ist insbesondere dann sinnvoll, wenn bei oraler Gabe ein großer Wirkstoffanteil bei der ersten Passage durch die Schleimhäute des Magen-Darm-Kanals metabolisiert bzw. durch die Leber zurückgehalten wird (First-Pass-Effekt) und/oder wenn der Wirkstoff eine niedrige Plasmahalbwertszeit aufweist. Das transdermale Darreichungssystem (*syn.* transdermales therapeutisches System, TTS) wird auf die Haut aufgeklebt und setzt den Wirkstoff frei, welcher dann über die Haut resorbiert wird. Dabei setzt die transdermale Verabreichung voraus, dass die verwendete Darreichungsform eine möglichst gleichmäßige und kontrollierte Abgabe des Wirkstoffs über einen längeren Zeitraum gewährleistet.

Um einen ausreichend hohen Plasmaspiegel zu ermöglichen bzw. einen gewünschten therapeutischen Effekt zu bewirken, sollten im Allgemeinen möglichst hohe Wirkstoff-Abgaberaten an bzw. durch die Haut erzielt werden. Die Wirkstoff-Abgaberate hängt üblicherweise zum einen von den Permeabilitätseigenschaften der Haut für einen betreffenden Wirkstoff und zum anderen von der Konzentration des Wirkstoffs in der Matrix des transdermalen therapeutischen Systems ab.

Um die Wirkstoffabgaberate zu verbessern, ist es daher üblich, die Wirkstoffkonzentration im Wirkstoffreservoir zu erhöhen, bis die Sättigungskonzentration erreicht wird, um auf diese Weise die thermodynamische Aktivität des Wirkstoffs zu steigern.

Allerdings hat eine solche Sättigungskonzentration häufig zur Folge, dass es während der Lagerung oder während der Applikationsdauer infolge des zumindest zeitweisen Überschreitens der Sättigungskonzentration des Wirkstoffs in der Matrix leicht zu einer Rekristallisation des Wirkstoffs kommen kann. Durch eine solche Rekristallisation nimmt die thermodynamische Aktivität des Wirkstoffs stark ab und als Folge hiervon auch die Wirkstoffabgaberate.

Es hat sich in der Praxis allerdings gezeigt, dass in vielen Fällen die Wirkstoff-Abgaberate eines TDS, insbesondere eine Permeationsrate des Wirkstoffs aus dem TDS in die Haut, trotz einer Erhöhung der Wirkstoffkonzentration im Wirkstoffreservoir und/oder gegebenenfalls eingesetzter Permeationsverstärker häufig zu gering ist oder eine Rekristallisation des Wirkstoffs zu beobachten ist.

Daraus kann sich einerseits der Nachteil ergeben, dass das transdermale therapeutische System in relativ kurzen Intervallen ersetzt werden muss. Um einen gewünschten therapeutischen Effekt zuverlässig zu erreichen, muss ein TDS daher häufig schon nach einem bis wenigen Tagen ausgetauscht werden. Damit wird die Applikationsdauer des transdermalen therapeutischen Systems unnötigerweise verkürzt, was sich nachteilig auf die Compliance auswirken kann. Dies ist gerade dann von Bedeutung, wenn eine Behandlung über einen längeren Zeitraum, wie beispielsweise die Behandlung von Krebs, über mehrere Monate notwendig ist.

Des Weiteren kann eine geringe Wirkstoff-Abgaberate bzw. Permeationsrate des Wirkstoffs auch dazu führen, dass nach einer vorgegebenen Applikationsdauer des transdermalen therapeutischen Systems ein Großteil des eingesetzten Wirkstoffs noch immer in der Matrix des Pflasters vorliegt (nachfolgend wird das TTS auch als Pflaster bezeichnet). Damit geht bei einem Wechsel des Pflasters unter Umständen ein bedeutender Anteil des Wirkstoffs ungenutzt verloren.

Es ist daher eine Aufgabe der vorliegenden Erfindung, ein transdermales therapeutisches System mit einem Aromatasehemmer bereitzustellen, aus welchem der Wirkstoff in ausreichender Menge kontinuierlich und kontrolliert aus dem TTS an die Haut abgegeben wird. Des Weiteren ist es eine Aufgabe der vorliegenden Erfindung, ein Verfahren zur Herstellung von TDS mit einem Aromatasehemmer bereitzustellen.

Diese Aufgabe wird erfindungsgemäß durch ein TDS mit einer Matrix umfassend einen Aromatasehemmer gemäß Anspruch 1, sowie durch ein Verfahren zur Herstellung des erfindungsgemäßen TDS gemäß Anspruch 7 gelöst. Des Weiteren wird die Aufgabe durch ein erfindungsgemäßes TDS zur Anwendung gemäß Anspruch 12 gelöst.

Die vorliegende Erfindung betrifft daher ein transdermales therapeutisches System, umfassend
(i) zumindest eine wirkstoffhaltige Matrix,
(ii) eine bevorzugt wirkstoffundurchlässige Rückschicht, und
(iii) eine optionale applikationsseitige Schutzfolie,
wobei die wirkstoffhaltige Matrix zumindest einen Wirkstoff aus der Klasse der Aromatasehemmer umfasst,
wobei der Wirkstoff aus der Klasse der Aromatasehemmer im Wesentlichen gelöst vorliegt, und
wobei die wirkstoffhaltige Matrix zumindest ein Polymer aus der Klasse der Silicone, insbesondere aus der Klasse der Amin-kompatiblen Silicone, umfasst.

In seiner einfachsten Ausführungsform umfasst das TDS zumindest eine wirkstoffhaltige Matrixschicht, welche horizontal in dem TDS angeordnet ist und mit welcher das TTS auf die Haut aufgebracht wird. Optional weist das TTS auf dessen Applikationsseite, d. h. auf der Seite, welche nach bestimmungsgemäßem Anbringen des TDS in Kontakt mit der Haut ist, eine Schutzschicht, vorzugsweise eine Schutzfolie, auf, welche auf der wirkstoffhaltigen Matrix aufgebracht ist und vor Applikation des TTS entfernt wird. Auf der der Applikationsseite abgewandten Seite ist das TDS von einer bevorzugt wirkstoffundurchlässigen Schicht, vorzugsweise von einer wirkstoffundurchlässigen Rückschicht, begrenzt.

Die Applikationsseite des TDS kann ganzflächig haftklebend ausgebildet sein, beispielsweise indem die wirkstoffhaltige Matrix selbst haftklebend oder mit einem selbsthaftenden Kleber ganzflächig beschichtet ist oder nur teilflächig haftklebend ausgebildet ist. Besonders bevorzugt kann die zumindest eine wirkstoffhaltige Matrixschicht selbstklebend ausgebildet sein.

Unter einer Matrix im Sinne der Erfindung ist bzw. sind dabei eine Matrix oder mehrere Matrices zu verstehen, deren Bestandteile zumindest ein Polymer aus der Klasse der Silicone (*syn.* Silikone) umfassen. Das erfindungsgemäße transdermale therapeutische System gibt den Wirkstoff aus der wirkstoffhaltigen Matrix an die Haut ab, wobei zumindest ein Teil des Wirkstoffs systemisch aufgenommen wird.

Erfindungsgemäß umfasst die zumindest eine wirkstoffhaltige Matrix des TDS wenigstens einen Wirkstoff, der ausgewählt ist aus der Klasse der Aromatasehemmer. Der Wirkstoff liegt bei den erfindungsgemäßen TDS im Wesentlichen gelöst in der zumindest einen wirkstoffhaltigen Matrix vor. Unter der Bezeichnung "gelöst" bzw. "in gelöster Form" wird dabei ein gelöster Wirkstoff aus der Klasse der Aromatasehemmer verstanden, welcher molekular dispers gelöst, vorzugsweise als homogenes einphasiges Gemisch in der (Polymer)matrix der Matrixschicht, vorliegt. Dies schließt nicht aus, dass die Konzentration des Wirkstoffs in der Matrixschicht über dessen Sättigungslöslichkeit liegt, so dass neben dem gelösten Wirkstoff auch ungelöster Wirkstoff, beispielsweise in amorpher oder kristalliner Form, vorliegen kann.

In den erfindungsgemäßen TDS liegt der Wirkstoff überwiegend in einer kristallfreien Form in der wirkstoffhaltigen Matrix vor. Allerdings kann der Wirkstoff, in geringen Mengen, auch komplexere Strukturen, z. B. bestehend aus zwei oder mehreren zusammengelagerten Wirkstoffmolekülen, umfassen. Weiterhin kann der überwiegend gelöst vorliegende Wirkstoff in der wirkstoffhaltigen Matrix, zu geringen Teilen, auch kristallartige Strukturen bzw. Wirkstoffkristalle umfassen. Insbesondere ist der im Wesentlichen gelöst vorliegende Wirkstoff aus der Klasse der Aromatasehemmer, insbesondere Anastrozol, dadurch gekennzeichnet, dass in der wirkstoffhaltigen Matrix kein Schmelzpunkt bzw. Schmelzbereich, insbesondere kein Schmelzpunkt bzw. Schmelzbereich von Wirkstoffkristallen, nachweisbar ist. Unter der Bezeichnung Schmelzpunkt bzw. Schmelzbereich ist die Temperatur zu verstehen, bei welcher ein Stoff, vorliegend insbesondere der Wirkstoff, von der festen Phase in die flüssige Phase übergeht. Eine Methode zur Bestimmung des Schmelzpunkts bzw. Schmelzbereichs ist dem Fachmann bekannt; so kann eine Bestimmung beispielsweise mit Hilfe der dynamischen Differenzkalorimetrie (DSC) erfolgen.

Vorzugsweise liegt der Wirkstoff aus der Klasse der Aromatasehemmer zu wenigstens 90 Flächen-%, bevorzugt zu wenigstens 95 Flächen-%, besonders bevorzugt zu wenigstens 97 Flächen-%, insbesondere zu wenigstens 98 Flächen-%, insbesondere bevorzugt zu wenigstens 99 Flächen-%, in kristallfreier Form in der wirkstoffhaltigen Matrix vor. Methoden zur Bestimmung der Angabe in Flächen-% sind dem Fachmann bekannt. So kann eine Bestimmung der Flächen-% beispielsweise mittels Mikroskopie erfolgen.

Erfindungsgemäß umfasst das TDS ein die Matrix bildendes Polymersystem auf Basis von Silicon, welche/welches den zumindest einen Wirkstoff aus der Klasse der Aromatasehemmer enthält. Unter einem Polymersystem auf Basis von Silicon bzw. einem Silicon-Kleber wird im Rahmen der vorliegenden Anmeldung eine Gruppe von synthetischen Polymeren verstanden, bei denen Siliciumatome über Sauerstoffatome miteinander verknüpft sind.

Vorzugsweise umfasst das Polymersystem der Matrix wenigstens 95 Gew.-% eines Polymers aus der Klasse der Silicone. Vorzugsweise umfasst das Polymersystem der Matrix wenigstens 96 Gew.-%, bevorzugt wenigstens 97 Gew.-%, besonders bevorzugt wenigstens 98 Gew.-%, insbesondere bevorzugt wenigstens 99 Gew.-%, zumindest eines Polymers aus der Klasse der Silicone.

Vorteilhafterweise kann mittels des erfindungsgemäßen transdermalen therapeutischen Systems erreicht werden, dass ein Wirkstoff, insbesondere ein Wirkstoff aus der Klasse der Aromatasehemmer, über einen bestimmten Zeitraum hinweg gleichmäßig und kontrolliert aus dem TDS abgegeben wird. Insbesondere auf Grund der erfindungsgemäßen Zusammensetzung des TDS kann die Permeationsrate des Wirkstoffs in die Haut im Vergleich zu bekannten transdermalen Darreichungssystemen deutlich erhöht und verlängert werden. Vorteilhafterweise kann dadurch erreicht werden, dass ein ausreichend hoher Plasmaspiegel des Wirkstoffs über einen bestimmten Zeitraum, insbesondere während einer Applikationsdauer des TDS von zumindest drei Tagen und/oder bis zu sieben Tagen, kontinuierlich aufrechterhalten werden kann, wodurch die Compliance des TDS in erheblichem Maße verbessert werden kann.

Weiterhin vorteilhaft kann auf Grund der erfindungsgemäßen Zusammensetzung des TDS und einer daraus resultierenden Erhöhung der Abgaberate des Wirkstoffs erreicht werden, dass ein im TDS verbleibender Restwirkstoffgehalt nach einer vorbestimmten Applikationsdauer des TDS deutlich reduziert ist gegenüber bekannten TTS. Vorzugsweise kann daher ein Restwirkstoffgehalt im TDS nach einer vorbestimmten Applikationsdauer bei höchstens etwa 50 Gew.-%, bevorzugt bei höchstens etwa 40 Gew.-%, besonders bevorzugt bei höchstens 30 Gew.-%, insbesondere bei höchstens etwa 20 Gew.-%, liegen. Das ist vorzugsweise der Fall für ein TDS, welches über einen Applikationszeitraum von mindestens 5 Tagen, bevorzugt von mindestens 6 Tagen, insbesondere von bis zu 7 Tagen, auf der Haut eines Patienten angewendet wurde. Dadurch lässt sich die Effizienz des transdermalen Darreichungssystems insgesamt verbessern und die Kosten signifikant erniedrigen.

Ein zusätzlicher Vorteil ergibt sich daraus, dass ein erfindungsgemäßes TDS über einen bestimmten Zeitraum eine verbesserte Stabilität im Hinblick auf die Rekristallisation des Wirkstoffs gewährleistet. Vorteilhaft kann das erfindungsgemäße TDS bei einer bevorzugten Temperatur von mindestens etwa 5 °C gelagert werden. Bevorzugt liegt die Temperatur für eine Lagerung der erfindungsgemäßen TDS bei etwa 5 °C bis zu etwa 25 °C oder bei einer Temperatur von etwa 5 °C bis zu etwa 30 °C oder bei einer Temperatur von etwa 5 °C bis zu etwa 40 °C, ohne dass hierbei ein wesentlicher Anteil des Wirkstoffs bzw. des Aromatasehemmers auskristallisiert. Insbesondere liegt eine bevorzugte Lagertemperatur der erfindungsgemäßen TDS bei etwa 5 °C bis zu etwa 25 °C.

So kann die erfindungsgemäße Zusammensetzung beispielsweise mehrere Monate, vorzugsweise mindestens 6 Monate, oder auch Jahre bei Raumtemperatur gelagert werden, ohne dass hierbei ein wesentlicher Anteil des Wirkstoffs auskristallisiert. Daher zeigt das erfindungsgemäße TDS auch nach Lagerung über mehrere Monate bzw. Jahre hinweg eine reproduzierbare und kontrollierte Abgabe des Wirkstoffs an bzw. durch die Haut. Dies ist insbesondere für die Verwendung der erfindungsgemäßen Zusammensetzung in Darreichungsformen wie transdermalen therapeutischen Systemen relevant, bei welchen eine Abgabe des Wirkstoffs aus einer Kristallform wesentlich verzögert bzw. verringert ist, weshalb eine wirksame Therapie nicht gewährleistet werden kann.

Das erfindungsgemäße TDS wird vorzugsweise zur Anwendung bei der Behandlung von Brustkrebs eingesetzt. Bevorzugt wird das TDS zur Behandlung eines Mammakarzinoms eingesetzt. Insbesondere kann das TDS zur adjuvanten, d. h. einer unterstützenden und/oder ergänzenden Behandlung, eines hormonabhängigen Mammakarzinoms verwendet werden. Insbesondere wird das TDS für die Behandlung eines Mammakarzinoms bei postmenopausalen Frauen eingesetzt.

Bevorzugt erfolgt die Behandlung mittels des Transdermalen Darreichungssystems dabei über einen jeweiligen Applikationszeitraum des TDS von zumindest drei Tagen bis zu sieben Tagen. Vorzugsweise kann das TDS für eine Applikationsdauer von 4 Tagen, bevorzugt von 5 Tagen, besonders bevorzugt von 6 Tagen, insbesondere von 7 Tagen, angewendet werden.

Die vorliegende Erfindung betrifft somit weiterhin die medizinische, tiermedizinische und/oder kosmetische Verwendung der erfindungsgemäßen Pflaster zur Abgabe von Wirkstoffen an bzw. durch die Haut eines menschlichen oder tierischen Körpers und/oder an eine Umgebung des Pflasters.

Des Weiteren betrifft die vorliegende Erfindung ein Verfahren zur Herstellung eines erfindungsgemäßen TDS, wobei das Verfahren folgende Schritte umfasst:
(i) Bereitstellen zumindest einer Matrix enthaltend einen Wirkstoff aus der Klasse der Aromatasehemmer und zumindest ein Polymer aus der Klasse der Silicone,
(ii) Aufbringen der zumindest einen wirkstoffhaltigen Matrix auf eine Schutzfolie zum Erhalt eines Laminats mit einer wirkstoffhaltigen Matrix,
(iii) Trocknen des Laminats umfassend die wirkstoffhaltige Matrix,
(iv) Kaschieren des die zumindest eine Matrix umfassenden Laminats aus Schritt (iii).

Unter "Bereitstellen" wird dabei sowohl eine Herstellung vor Ort als auch ein Zuliefern einer wirkstoffhaltigen Matrix verstanden. Hierbei kann eine wirkstoffhaltige Matrix bereits mit einer die Applikationsseite der wirkstoffhaltigen Matrix bedeckenden Schutzfolie versehen sein, welche im weiteren Verlauf der Herstellung auf dieser verbleibt oder optional in einem oder mehreren Herstellschritten durch eine alternative Schutzfolie ersetzt werden kann.

Erfindungsgemäß umfasst die zumindest eine wirkstoffhaltige Matrix gemäß Schritt (i) wenigstens einen Wirkstoff, der ausgewählt ist aus der Klasse der Aromatasehemmer.

Vorzugsweise liegt der Wirkstoff auch in der wirkstoffhaltigen Matrix im Wesentlichen gelöst bzw. in Lösung vor, d. h. in molekular dispers gelöster Form, vorzugsweise als homogenes einphasiges Gemisch. Dies schließt wiederum nicht aus, dass die Konzentration des Wirkstoffs in der wirkstoffhaltigen Matrix gemäß Schritt (i) über dessen Sättigungslöslichkeit liegt, so dass neben dem gelösten Wirkstoff auch ungelöster Wirkstoff, beispielsweise in amorpher oder kristalliner Form, vorliegen kann.

Unter einer Lösung im Sinne der vorliegenden Erfindung wird hierbei ein Gemisch eines Solvens und eines Solvats verstanden, wobei das Solvat, also der Wirkstoff, im Wesentlichen molekular dispers vorliegt, d. h. eine Partikelgröße von unter 1 nm aufweisen kann. Jedoch kann eine Lösung auch kolloidal dispers gelöste Partikel mit einer Größe im Bereich von 1 nm bis 1 µm und/oder grob dispers gelöste Partikel mit einer Größe von über 1 µm aufweisen. Bevorzugt liegt jedoch der Wirkstoff auch in der wirkstoffhaltigen Matrix gemäß Schritt (i) im Wesentlichen in molekular dispers gelöster Form vor.

Gemäß einer bevorzugten Ausführungsform können flächige Wirkstoffkerne, z. B. wirkstoffhaltige Matrixkerne, vor oder nach Aufbringen auf eine Schutzfolie aus dem Laminat mit einer wirkstoffhaltigen Matrix gestanzt und im weiteren Verlauf dem erfindungsgemäßen Herstellverfahren unterzogen werden. Unter einem Wirkstoffkern ist hierbei eine flächige wirkstoffhaltige Matrix zu verstehen, welche vorteilhaft auf deren Rückseite und/oder auf deren Applikationsseite eine Rückschicht bzw. eine Schutzfolie umfasst und welche mit Hilfe eines Schneidwerkzeugs, insbesondere eines Stanzwerkzeugs, aus dem oben genannten wirkstoffhaltigen Laminat geschnitten, insbesondere gestanzt, wird. Insbesondere bevorzugt werden flächige Wirkstoffkerne aus einem Laminat, welches die wirkstoffhaltige Matrix und eine Schutzfolie umfasst, gestanzt.

Schließlich umfasst die vorliegende Erfindung ein transdermales therapeutisches System, welches nach einem vorstehend beschriebenen Verfahren erhältlich ist.

Weitere besonders vorteilhafte Ausgestaltungen und Weiterbildungen der Erfindung ergeben sich aus den abhängigen Ansprüchen sowie der nachfolgenden Beschreibung, wobei die Patentansprüche einer bestimmten Kategorie auch gemäß den abhängigen Ansprüchen einer anderen Kategorie weitergebildet sein können und Merkmale verschiedener Ausführungsbeispiele zu neuen Ausführungsbeispielen kombiniert werden können.

Die vorliegende Erfindung kann ebenfalls TTS mit einem komplexeren Aufbau, wie beispielsweise mit zwei oder mehr Matrixschichten unterschiedlicher Zusammensetzung und Klebeeigenschaften, umfassen. Das erfindungsgemäße transdermale Darreichungssystem kann also zusätzlich zu der zumindest einen wirkstoffhaltigen Matrix noch eine oder mehrere Matrixschichten umfassen. Bevorzugt können die mehreren Matrixschichten jeweils haftklebend ausgebildet sein und einander horizontal aufgelagert sein. Die zusätzlichen Matrixschichten können wirkstoffhaltig oder wirkstofffrei ausgebildet sein.

Sofern das TDS mehr als nur eine wirkstoffhaltige Matrixschicht umfasst, können die jeweiligen Schichten denselben oder unterschiedliche Wirkstoffe umfassen. Ein solcher Wirkstoff kann dabei eine pharmazeutisch wirksame Substanz oder auch ein kosmetischer Wirkstoff und/oder ein Zusatzstoff und/oder ein Nährstoff oder ein Nahrungsergänzungsmittel sein. Eine Konzentration des Wirkstoffs in den jeweiligen Matrixschichten kann im Wesentlichen gleich oder auch andersartig sein. Das bedeutet, die TDS können zusätzlich zu dem zumindest einen Wirkstoff aus der Klasse der Aromatasehemmer grundsätzlich alle Wirkstoffe oder Wirkstoffkombinationen aufweisen. Grundsätzlich kann die Rezeptur der jeweiligen Matrixschichten also identisch oder unterschiedlich sein. Bevorzugt weist das erfindungsgemäße TDS jedoch nur einen Wirkstoff aus der Klasse der Aromatasehemmer auf.

Sofern das TDS mehr als nur eine wirkstoffhaltige Matrixschicht umfasst, wird die Matrix, in welcher der Aromatasehemmer im Wesentlichen gelöst vorliegt, nachfolgend auch als erste Matrix des TDS bezeichnet. Eine weitere, der Rückschicht (*syn.* Backingfolie) des TDS zugewandte und/oder der Haut zugewandte (zweite) Matrix des TDS kann wirkstoffhaltig oder wirkstofffrei ausgebildet sein. Vorzugsweise umfasst auch die (zweite) Matrix einen Wirkstoff, der ausgewählt ist aus der Klasse der Aromatasehemmer. Der Wirkstoff kann überwiegend gelöst in der (zweiten) Matrix enthalten sein. Besonders bevorzugt kann der Wirkstoff allerdings als Suspension in der (zweiten) Matrix vorliegen.

Darüber hinaus kann das TDS, wie erwähnt, grundsätzlich noch weitere Schichten bzw. Matrices umfassen, die jeweils wirkstoffhaltig oder wirkstofffrei ausgebildet sein können. Beispielsweise könnte eine dritte Schicht zwischen der (zweiten) Matrix und der Rückschicht des TDS angeordnet sein. Vorzugsweise kann die (dritte) Matrix einen Wirkstoff umfassen, der ausgewählt ist aus der Klasse der Aromatasehemmer. Der Wirkstoff kann überwiegend gelöst in der (dritten) Matrix angeordnet sein. Besonders bevorzugt allerdings kann der Wirkstoff als Suspension in der (dritten) Matrix vorliegen.

Für den Fall, dass das TDS mehr als nur eine Matrix umfasst, kann eine Matrixschicht des TDS, bevorzugt eine zweite wirkstoffhaltige Matrix, von der ersten Matrix vorteilhaft durch eine zwischen die erste und die zweite Matrix aufgebrachte Kontrollmembran getrennt sein. So kann beispielsweise die der Rückschicht zugewandte zweite wirkstoffhaltige Matrix, die den Wirkstoff in Form einer Suspension enthält, durch eine Kontrollmembran von der ersten wirkstoffhaltigen Applikationsschicht getrennt sein, welche den Wirkstoff überwiegend in kristallfreier Form enthält. Die jeweiligen Matrixschichten sind vorzugsweise ganzflächig selbsthaftend ausgebildet.

In einem entsprechenden Herstellverfahren wird zwischen die (zweite) wirkstoffhaltige Matrix und die wirkstoffhaltige Applikationsschicht eine Kontrollmembran eingebracht. Eine Wirkstoffdiffusion wird dabei durch Poren in der Kontrollmembran ermöglicht, welche einen Porendurchmesser von bevorzugt mindestens etwa 0,05 µm, besonders bevorzugt von mindestens etwa 0,075 µm, insbesondere von mindestens etwa 0,1 µm, aufweist.

Insbesondere basiert eine bevorzugte Kontrollmembran auf einem Polymer ausgewählt aus der Gruppe umfassend Polyolefine, Olefin-Copolymerisate, Polyester, Co-Polyester, Polyamide, Co-Polyamide, Polyurethane und dergleichen. Vorzugsweise kann das Polymer ausgewählt sein aus Polyethylen, bevorzugt aus Polyethylenterephthalat. Besonders bevorzugt kann das Polymer ausgewählt sein aus Polypropylen. Eine besonders bevorzugte Kontrollmembran umfasst ein Polypropylen und kann beispielsweise unter der Bezeichnung Celgard von der Firma Azelis (Deutschland) erworben werden.

Zur besseren Verdeutlichung der der Erfindung zugrunde liegenden Idee wird die Erfindung nachfolgend, ohne eine Beschränkung darauf, sofern nicht anders erwähnt, anhand eines besonders bevorzugten TDS mit nur einer wirkstoffhaltigen Matrix beschrieben, wobei der Wirkstoff in der Matrix der Applikationsschicht im Wesentlichen in Lösung vorliegt.

Die Menge des Wirkstoffs, der in der Zusammensetzung enthalten sein soll, variiert in Abhängigkeit von der gewünschten therapeutischen Wirkung und der Zeitspanne, während der das TDS für eine Therapie sorgen soll. Bei den meisten Wirkstoffen ist der Durchtritt durch die Haut der geschwindigkeitsbestimmende Schritt für deren Abgabe. Die Menge des Wirkstoffs und die Freisetzungsgeschwindigkeit werden also typischerweise so gewählt, dass man eine transdermale Abgabe erhält, die während einer längeren Zeitspanne durch eine Zeitabhängigkeit von im Wesentlichen nullter Ordnung gekennzeichnet ist.

In einer bevorzugten Ausführungsform der Erfindung ist der Wirkstoff der zumindest einen wirkstoffhaltigen Matrix ausgewählt aus Anastrozol. Alternativ oder zusätzlich können die zumindest eine wirkstoffhaltige Matrix oder weitere Matrices einen Aromatasehemmer umfassen, der ausgewählt ist aus Letrozol, Exemestan, Vorozol, Formestan, Fadrozol, Aminoglutethimid oder Testolacton.

Vorzugsweise variiert ein Gehalt an Aromatasehemmer in der zumindest einen wirkstoffhaltigen Matrix des transdermalen Darreichungssystems in einem Bereich von mindestens etwa 1 Gew.-% bis höchstens etwa 30 Gew.-%. Bevorzugt liegt ein Gehalt an Anastrozol in der zumindest einen wirkstoffhaltigen Matrix in einem Bereich von wenigstens in etwa 5 Gew.-% und höchstens 15 Gew.-%, besonders bevorzugt im Bereich von wenigstens etwa 7 Gew.-% und höchstens 10 Gew.-%. Sofern die wirkstoffhaltige Matrix eine Mischung unterschiedlicher Aromatasehemmer umfasst, kann ein Gesamtgehalt der eingesetzten Aromatasehemmer in den zuvor definierten Grenzen liegen. Insofern nicht anders hingewiesen, beziehen sich die Angaben in Gew.-% vorliegend auf die getrocknete Matrix.

Der Wirkstoff kann in verschiedenen Formen in der Matrix enthalten sein, je nachdem, welche Form die optimale Abgabeeigenschaft des Wirkstoffs aus dem TDS bzw. der Matrix ergibt. Im Falle des Aromatasehemmers bzw. anderer pharmazeutisch wirksamer Substanzen können diese in Form der freien Base oder Säure oder in Form von Salzen, Estern, Hydraten oder anderen pharmakologisch akzeptablen Derivaten oder als Komponenten von molekularen Komplexen vorliegen. Besonders bevorzugt liegt der Aromatasehemmer, insbesondere Anastrozol, in Form der freien Base in der wirkstoffhaltigen Matrix vor.

In einer bevorzugten Ausführungsform der Erfindung umfasst das Polymersystem der zumindest einen wirkstoffhaltige Matrix, gegebenenfalls auch das Polymersystem der weiteren Matrixschichten, ein selbstklebendes Polymer aus der Klasse der Silicone. Das zumindest eine Polymer der wirkstoffhaltigen Matrix ist vorzugsweise ausgewählt aus der Klasse der Amin-kompatiblen Silicone. Besonders bevorzugt ist das Polymer ausgewählt aus der Gruppe der Polysiloxane. Das Polymersystem umfasst also eine Anzahl von miteinander verbundenen Siloxaneinheiten. Dabei kann das Polymersystem eine Anzahl von cyclischen Polysiloxanen, und/oder linearen Polysiloxanen, und/oder vernetzten Polysiloxanen, und/oder verzweigten Polysiloxanen, umfassen. Vorzugsweise umfasst das Polymersystem eine Anzahl von linearen Polysiloxanen, und/oder vernetzten Polysiloxanen, und/oder verzweigten Polysiloxanen.

Insbesondere ist das zumindest eine Polymer der wirkstoffhaltigen Matrix, bzw. der weiteren Matrixschichten ausgewählt aus der Gruppe von BIO-PSA 7-4101 Silicone Adhesive, BIO-PSA 7-4201 Silicone Adhesive, BIO-PSA 7-4301 Silicone Adhesive, BIO-PSA 7-4102 Silicone Adhesive, BIO-PSA 7-4202 Silicone Adhesive oder BIO-PSA 7-4302 Silicone Adhesive (Hersteller Dow Corning, USA) oder Mischungen daraus.

Neben dem zuvor erläuterten Polymersystem auf Basis von Silicon kann die zumindest eine wirkstoffhaltige Matrix, gegebenenfalls auch die weiteren Matrixschichten, in geringen Mengen noch weitere Komponenten umfassen, die gewöhnlich für transdermale therapeutische Systeme verwendet werden. Bevorzugt können einer Matrix z. B. Klebrigmacher zugesetzt werden, um zu einer selbstklebenden (d. h. haftklebenden) wirkstoffhaltigen Matrix zu gelangen, als Alternative oder zusätzlich zu der zuvor erwähnten hautseitigen haftklebenden Schicht.

Die Matrix kann zusätzlich auch weitere Polymere umfassen, die bei der Herstellung von transdermalen Systemen eingesetzt werden und physiologisch unbedenklich sind, wie z. B. Homo- und Copolymere von (Meth)acrylaten, Polyvinylether, Polyisoprenkautschuke, Styrol-Butadien-Copolymere oder Styrol-Butadien-Styrol-Copolymere. Von den (Meth)acrylat-Copolymeren können beispielsweise die Copolymere von Alkylacrylaten und/oder Alkylmethacrylaten und weiteren ungesättigten Monomeren genannt werden, wie Acrylsäure, Methacrylsäure, Acrylamid, Dimethylacrylamid, Dimethylaminoethylacrylamid, Acrylnitril und/oder Vinylacetat.

Bevorzugt kann das die zumindest eine Matrix bildende Polymer zusätzlich ein Lösemittel umfassen, welches ausgewählt ist aus Ethylacetat reinst Ph. Eur. (AppliChem, Deutschland) und/oder Heptan, reinst (AppIChem, Deutschland). Besonders bevorzugt umfasst die wirkstoffhaltige Matrix jedoch Ethylacetat.

Vorzugsweise liegt ein Restanteil von Ethylacetat und/oder Heptan, insbesondere von Ethylacetat, in der zumindest einen wirkstoffhaltigen Matrix des TTS, in der der Wirkstoff überwiegend in gelöster Form vorliegt, bei weniger als 5000 ppm, bevorzugt bei weniger als 4000 ppm, besonders bevorzugt bei weniger als 3000 ppm. Die jeweiligen Angaben beziehen sich dabei auf ein anwendungsbereites TDS, d. h. auf ein TDS, in welchem die wirkstoffhaltige Matrix in getrockneter Form vorliegt. Besonders vorteilhafte Zusammensetzungen der zumindest einen wirkstoffhaltigen Matrix werden zu einem späteren Zeitpunkt erläutert.

Bevorzugt können in der zumindest einen wirkstoffhaltigen Matrix zusätzlich Hilfsstoffe, insbesondere Permeationsverstärker, zum Einsatz kommen. Bevorzugt kann die zumindest eine wirkstoffhaltige Matrix einen Hilfsstoff umfassen, der ausgewählt ist aus der Gruppe der Milchsäureester. Besonders bevorzugt kann die zumindest eine wirkstoffhaltige Matrix einen Gehalt an Lauryllactat umfassen. Ein solcher Permeationsverstärker kann z. B. unter der Bezeichnung Schercemol LL Ester des Herstellers Lubrizol Advanced Materials (USA) erworben werden.

Insbesondere bewirkt ein Gehalt eines oder mehrerer der bevorzugten Permeationsverstärker eine kontrollierte und anhaltende Wirkstoffabgabe aus dem bevorzugten transdermalen therapeutischen System, bevorzugt über einen Abgabezeitraum von bis zu 4, bis zu 5, bis zu 6 oder bis zu 7 Tagen, insbesondere von bis zu 7 Tagen. Vorteilhaft liegt ein Gehalt eines Permeationsverstärkers in der wirkstoffhaltigen Matrix in einem Bereich von wenigstens 0,1 Gew.-% und/oder höchstens 15 Gew.-%. Bevorzugt kann ein Gehalt eines Permeationsverstärkers bei zumindest etwa 0,5 Gew.-% und/oder bei nicht mehr als etwa 10 Gew.-% liegen.

Als weitere Komponente umfasst ein bevorzugtes erfindungsgemäßes TDS zumindest eine wirkstoffhaltige Matrix, gegebenenfalls auch weitere Matrixschichten, mit einem Gehalt an Siliziumdioxid. Ein vorteilhafter Gehalt an Siliziumdioxid in der zumindest einen Matrix liegt dabei bevorzugt in einem Bereich von mindestens etwa 1 Gew.-%, und/oder von höchstens etwa 10 Gew.-%. Besonders bevorzugt liegt ein Gehalt an Siliziumdioxid in der zumindest einen wirkstoffhaltigen Matrix bei wenigstens etwa 2,5 Gew.-%, und/oder bei höchstens etwa 7,5 Gew.-%, insbesondere bei etwa 5 Gew.-%.

Bevorzugt liegt das Siliziumdioxid in amorpher Form vor. Insbesondere weist ein Siliziumdioxid eine mittlere Primärteilchengröße von 5 bis 50 nm sowie eine spezifische Oberfläche von 50 bis 500 m²/g auf. Ein solches Siliziumdioxid ist beispielsweise unter der Bezeichnung Aerosil® 200 Pharma von der Firma Evonik Degussa (Deutschland) käuflich erhältlich.

Ein bevorzugtes TDS kann als Permeationsverstärker weiterhin einen Gehalt an Dipropylenglykol umfassen. Vorzugsweise kann der Gehalt an Dipropylenglykol in der zumindest einen wirkstoffhaltigen Matrix zumindest etwa 0,5 Gew.-%, und/oder höchstens etwa 5 Gew.-%, betragen. Bevorzugt kann der Gehalt an Dipropylenglykol zumindest etwa 1 Gew.-%, und/oder höchstens etwa 3 Gew.-%, besonders bevorzugt etwa 1,5 bis etwa 2 Gew.-%, betragen.

Vorteilhafterweise ist es auf Grund der besonderen Zusammensetzung des TDS möglich, dass auf die zusätzliche Verwendung von Kristallisationsinhibitoren bzw. Lösungsvermittlern und Antioxidantien verzichtet werden kann, wobei der Wirkstoff in der zumindest einen wirkstoffhaltigen Matrix des TDS dennoch überwiegend in kristallfreier Form vorliegt und somit eine ausreichende, kontrollierte und kontinuierliche Wirkstoff-Abgaberate aus dem TDS erreicht werden kann. In gleicher Weise kann auch bei weiteren Schichten des TDS auf den Einsatz von Kristallisationsinhibitoren bzw. Antioxidantien verzichtet werden.

Weiterhin vorteilhaft kann daher auf Grund der besonderen Zusammensetzung des TDS das Risiko von Hautirritationen gegenüber herkömmlichen TDS reduziert werden. Des Weiteren kann vorteilhaft die Problematik einer möglichen Inkompatibilität zwischen dem Wirkstoff und zusätzlich eingesetzten Kristallisationsinhibitoren, Lösungsvermittlern und/oder Antioxidantien vermieden werden.

Bevorzugt kann die zumindest eine wirkstoffhaltige Matrix des transdermalen Darreichungssystems so ausgebildet sein, dass sie frei ist von Kristallisationsinhibitoren. Besonders bevorzugt kann die wirkstoffhaltige Matrix, in der der Wirkstoff überwiegend gelöst vorliegt, so ausgebildet sein, dass sie kein Polyvinylpyrrolidon (PVP), insbesondere kein Crospovidon (Ph.Eur. 7. Edition Supplement 7.4, Type B) oder Copovidon (Kollidon® CL-M oder Kollidon® VA64), enthält.

Eine vorteilhafte Zusammensetzung einer Matrix umfassend einen Aromatasehemmer mit einem Polymersystem auf Basis von Silicon umfasst
(i) 1 bis 30 Gew.-% eines Aromatasehemmers,
(ii) 15 bis 98 Gew.-% eines Polymersystems auf Basis von Silicon,
(iii) 0,1 bis 10 Gew.-% eines Milchsäureesters,
(iv) gegebenenfalls 1 bis 10 Gew.-% Siliziumdioxid, und
(v) gegebenenfalls 5 bis 15 Gew.-% Dipropylenglykol.

Bevorzugt umfasst eine Matrix umfassend einen Aromatasehemmer mit einem Polymersystem auf Basis von Silicon
(i) 2 bis 15 Gew.-% des Aromatasehemmers, insbesondere 3 bis 13 Gew.-% Anastrozol,
(ii) 40 bis 94 Gew.-% des Polymersystems auf Basis von Silicon, insbesondere 50 bis 85 Gew.-% zumindest eines Polymers aus der Klasse der Silicone,
(iii) 0,5 bis 7,5 Gew.-% des Milchsäureesters
(iv) 2 bis 9 Gew.-% Siliziumdioxid, und
(v) gegebenenfalls 5 bis 15 Gew.-% Dipropylenglykol.

Besonders bevorzugt sind die Bestandteile einer Matrix umfassend einen Aromatasehemmer auf Silicon-Basis ausgewählt aus
(i) 4 bis 12 Gew.-% Anastrozol,
(ii) 60 bis 80 Gew.-% zumindest eines Polymers aus der Klasse der Silicone,
(iii) 1 bis 5 Gew.-% Lauryllactat,
(iv) 3 bis 8 Gew.-% Siliziumdioxid,
(v) 0,1 bis 5 Gew.-% Dipropenylglykol,
(vi) gegebenenfalls bis zu 5000 ppm Ethylacetat, und
(vii) gegebenenfalls bis zu 3000 ppm Heptan.

Insbesondere sind die Bestandteile einer Matrix umfassend einen Aromatasehemmer auf Silicon-Basis ausgewählt aus
(i) 5 bis 11 Gew.-% Anastrozol,
(ii) 65 bis 75 Gew.-% zumindest eines Polymers aus der Klasse der Silicone,
(iii) 2 bis 4 Gew.-% Lauryllactat,
(iv) 3,5 bis 7 Gew.-% Siliziumdioxid,
(v) 0,5 bis 3 Gew.-% Dipropenylglykol,
(vi) gegebenenfalls bis zu 3000 ppm Ethylacetat, und
(vii) gegebenenfalls bis zu 1000 ppm Heptan.

Insbesondere bevorzugt sind die Bestandteile einer Matrix umfassend einen Aromatasehemmer auf Silicon-Basis ausgewählt aus
(i) 6 bis 10 Gew.-% Anastrozol,
(ii) 68 bis 74 Gew.-% zumindest eines Polymers aus der Klasse der Silicone,
(iii) 2,5 bis 3,5 Gew.-% Lauryllactat,
(iv) 4 bis 6 Gew.-% Siliziumdioxid,
(v) 1 bis 2,5 Gew.-% Dipropenylglykol,
(vi) gegebenenfalls bis zu 2500 ppm Ethylacetat.

Insbesondere liegt ein bevorzugter Gehalt an Anastrozol bei mindestens etwa 8 Gew.-% bis zu höchstens etwa 10 Gew.-%, insbesondere bevorzugt bei etwa 9 Gew.-%. Ein Gehalt des zumindest einen Polymers aus der Klasse der Silicone liegt bei zumindest etwa 69 Gew.-%, bevorzugt bei zumindest etwa 70 Gew.-%, besonders bevorzugt bei zumindest etwa 71 Gew.-%, und/oder höchstens bei etwa 73 Gew.-%, insbesondere bei etwa 72 Gew.-%. Ein Gehalt an Lauryllactat beträgt höchstens 5 Gew.-%, bevorzugt höchstens 4 Gew.-%, insbesondere 3 Gew.-%. Ein Gehalt an Dipropenylglykol beträgt zumindest 0,5 Gew.-%, und/oder höchstens 5 Gew.-%, insbesondere 1,5 bis 2 Gew.-%, und ein bevorzugter Gehalt an Siliziumdioxid beträgt 5 Gew.-%. Ein Gehalt an Ethylacetat und/oder Heptan, insbesondere von Ethylacetat, beträgt bis zu 1000 ppm, insbesondere bis zu 2000 ppm.

Um eine Migration von Wirkstoff aus der zumindest einen Matrix zu verhindern, weist ein bevorzugtes transdermales Darreichungssystem vorteilhaft eine okklusive Rückschicht auf. Als Rückschichten eines erfindungsgemäßen TDS werden üblicherweise sogenannte Backingfolien aus beispielsweise Polyester mit einer Stärke von vorzugsweise mindestens etwa 5 µm, besonders bevorzugt von mindestens etwa 10 µm, insbesondere von mindestens etwa 20 µm, insbesondere bevorzugt von mindestens etwa 30 µm, verwendet. Höchstens weist dabei eine Backingfolie aus beispielsweise Polyester eine Stärke von bevorzugt bis zu etwa 200 µm, besonders bevorzugt bis zu etwa 150 µm, insbesondere von bis zu etwa 100 µm, insbesondere bevorzugt von bis zu etwa 50 µm, am meisten bevorzugt von bis zu etwa 40 µm, auf. Solche Backingfolien sind flexibel und können sich gegebenenfalls um die Ränder der Matrixschicht, d.h. um die in laterale Richtungen weisenden Seitenflächen der wirkstoffhaltigen Matrix legen und diese abdecken.

Bevorzugt basiert eine Rückschicht, insbesondere eine okklusive Rückschicht, auf einem Polymer ausgewählt aus der Gruppe bestehend aus Polyolefinen, Olefin-Copolymerisaten, Polyestern, Co-Polyestern, Polyamiden, Co-Polyamiden, Polyurethanen und dergleichen. Als Beispiele für geeignete Materialien können Polyester und hiervon insbesondere Polyethylenterephthalate (PET) als auch Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate, wie beispielsweise Acrylnitril-Butadien-Styrol-Terpolymere, oder Ethylen-Vinylacetat-Copolymerisate. Ein bevorzugtes Material einer Rückschicht ist ausgewählt aus der Gruppe der Polyolefine, besonders bevorzugt aus einem Polyethylen. Eine solche Rückschicht kann beispielsweise unter der Bezeichnung CoTran 9720 von der Firma 3M (USA) erworben werden.

Auch kann eine Rückschicht eines erfindungsgemäßen TDS eine Deckschicht bzw. ein Overtape umfassen, welches seitlich über die Ränder der zumindest einen wirkstoffhaltigen Matrix hinausragt und so eine verbesserte Adhäsion des erfindungsgemäßen TDS auf der Haut ermöglichen kann. Eine bevorzugte Deckschicht bzw. ein bevorzugtes Overtape ist dabei okklusiv ausgebildet.

Insbesondere umfasst ein Overtape eine wirkstofffreie Kleberschicht und eine Overtape-Folie, wobei eine Overtape-Folie bevorzugt ein Polymer ausgewählt aus der Gruppe der Polyolefine, Olefin Copolymerisate, Polyester, Co-Polyester, Polyamide, Co-Polyamide, Polyurethane und dergleichen umfasst. Ein bevorzugtes Material eines Overtapes ist ausgewählt aus einem Polyester, besonders bevorzugt aus einem Polyethylenterephthalat.

Bei einer Ausführungsform des erfindungsgemäßen TTS kann wie oben erwähnt die wirkstoffhaltige Matrix oder eine wirkstoffhaltige Applikationsschicht mit einer in der Fachsprache auch als Releaseliner bezeichneten abziehbaren Schutzfolie abgedeckt sein. Bei der Aufbewahrung des TTS wird hierdurch die wirkstoffhaltige Matrix bzw. die Applikationsschicht vor mechanischen Einflüssen und/oder einem unerwünschten Luftzutritt wirksam geschützt. Durch die Unterbindung eines unerwünschten Luftzutritts wird einer Zersetzung von in der Matrix enthaltenen Wirkstoffen, insbesondere von sauerstoffempfindlichen Wirkstoffen, vorgebeugt und so die Lager- bzw. Langzeitstabilität des Pflasters verbessert. Zur Applikation des Pflasters wird dann vor der Befestigung des Systems auf der Haut zunächst die Schutzfolie des Pflasters abgezogen. Zum besseren Greifen und um dadurch ein Ablösen der Schutzfolie zu erleichtern, ragt die Schutzfolie bei vorteilhaften Pflastern über den Rand des restlichen Pflasters hinaus.

Ein geeignetes Flächengewicht der wirkstoffhaltigen getrockneten Matrix kann sich in einem für transdermale therapeutische Systeme üblichen Bereich bewegen. Jedoch liegt ein bevorzugtes Flächengewicht der zumindest einen wirkstoffhaltigen Matrix bei mindestens etwa 20 mg/10cm², bevorzugt bei mindestens etwa 40 mg/10 cm², besonders bevorzugt bei mindestens etwa 60 mg/10 cm², insbesondere bevorzugt bei etwa 80 mg/10 cm², und/oder vorzugsweise bei bis zu etwa 120 mg/10 cm², bevorzugt bei bis zu etwa 100 mg/10 cm², besonders bevorzugt bei bis zu etwa 90 mg/10 cm².

Insofern das erfindungsgemäße TDS mehr als eine Matrix aufweist, so bewegt sich das Flächengewicht einer ersten bzw. weiteren Matrix bevorzugt bei mindestens etwa 20 mg/10 cm², besonders bevorzugt bei mindestens etwa 30 mg/10 cm², insbesondere bei mindestens etwa 40 mg/10 cm². Höchstens liegt das bevorzugte Flächengewicht einer ersten bzw. einer weiteren Matrix bei bis zu etwa 60 mg/10 cm², besonders bevorzugt bei bis zu etwa 50 mg/10 cm², insbesondere bei bis zu etwa 40 mg/10 cm². Jedoch muss dabei eine erste oder weitere Matrix nicht dasselbe Flächengewicht aufweisen, vielmehr kann eine erste Matrix beispielsweise ein Flächengewicht von mindestens etwa 20 mg/10 cm² bis zu etwa 40 mg/10 cm² und eine weitere Matrix ein Flächengewicht von beispielsweise mindestens etwa 40 mg/10 cm² bis zu etwa 60 mg/10 cm² aufweisen.

Eine Abstimmung der Flächengewichte auf die Gesamtschichtdicke bzw. das Gesamtflächengewicht des erfindungsgemäßen TDS erscheint gerade dann sinnvoll, wenn eine erste initiale Abgabe eines Wirkstoffs aus einer Applikationsschicht mit einem Wirkstoffgehalt mit einer zweiten länger andauernden Wirkstoffabgabe aus einer der Rückschicht zugewandten Matrixschicht abgestimmt werden soll. Ebenfalls ist bei der Auswahl des Flächengewichts der Einfluss auf die Trageeigenschaften des TDS zu berücksichtigen.

Zur Herstellung eines bevorzugten TDS kann die zumindest eine den Wirkstoff enthaltende Matrix so bereitgestellt werden, dass ein Feststoffanteil des Polymersystems auf Basis von Silicon, insbesondere vor einer optionalen Trocknung der Matrix, in einem Bereich von wenigstens etwa 50 Gew.-%, und/oder höchstens etwa 70 Gew.-%, liegt. Bevorzugt wird ein Feststoffanteil des Polymersystems auf einen Wert von wenigstens etwa 55 Gew.-%, bevorzugt von wenigstens etwa 58 Gew.-%, und/oder von höchstens etwa 65 Gew.-%, insbesondere von etwa 60 Gew.-%, eingestellt. Bevorzugt kann der Feststoffgehalt des Polymersystems, bzw. des Silicon-Klebers mittels Ethylacetat und/oder Heptan eingestellt werden. Besonders bevorzugt wird der Feststoffgehalt des Klebers mittels Ethylacetat eingestellt. Es hat sich gezeigt, dass die Verwendung von Ethylacetat zur Einstellung des Feststoffgehalts die Löslichkeit von Anastrozol in dem vorteilhaften TDS verbessern kann.

Vorzugsweise wird die zumindest eine den Wirkstoff enthaltende Matrix so bereitgestellt, dass die (nicht getrocknete) wirkstoffhaltige Matrix mit deren gesamten Bestandteilen einen Feststoffgehalt von wenigstens etwa 35 Gew.-%, und/oder höchstens von etwa 55 Gew.-%, hat. Bevorzugt wird der Feststoffgehalt der (nicht getrockneten) Matrix auf einen Wert von zumindest 40 Gew.-%, und/oder höchstens von 55 Gew.-%, insbesondere von etwa 45 Gew.-%, eingestellt.

Gemäß einer bevorzugten Ausführungsform eines Herstellungsverfahrens kann die bereitgestellte den Wirkstoff enthaltende zumindest eine Matrix kontrolliert getrocknet werden. Dazu kann die bereitgestellte Matrix zum Erhalt eines Laminats mit einer wirkstoffhaltigen Matrix vor dem Trocknungsvorgang optional auf eine Folie aufgebracht werden. Bevorzugt kann die Aufbringung der bereitgestellten Matrix auf die Folie so erfolgen, dass die Matrix nach der Trocknung ein gewünschtes Flächengewicht hat, beispielsweise ca. 80 g/m². Dazu kann die bereitgestellte Matrix z. B. auf einer einseitig fluoropolymerisierten Polyester-Folie (PETP) mit einer Stärke von etwa 75 µm durch Ausstreichen aufgebracht werden. Eine derartige Folie ist z. B. von dem Hersteller 3M (USA) erhältlich.

Die Trocknung der zumindest einen wirkstoffhaltigen Matrix erfolgt bei einer Temperatur in einem Bereich von wenigstens etwa 30 °C und/oder höchstens etwa 120 °C. Bevorzugt erfolgt die Trocknung bei zwei oder mehr unterschiedlichen Temperaturen. Vorzugsweise kann eine erste Trocknungstemperatur in einem Bereich von zumindest 30 °C und/oder höchstens 50 °C, insbesondere bei etwa 40 °C, liegen. Eine zweite Trocknungstemperatur kann in einem Bereich von zumindest 50 °C und/oder höchstens 70 °C, insbesondere bei etwa 60 °C, liegen. Eine dritte Trocknungstemperatur kann in einem Bereich von zumindest 80 °C und/oder höchstens 120 °C, insbesondere bei etwa 100 °C, liegen. Vorzugsweise liegt auch eine vierte Trocknungstemperatur im Bereich von zumindest 80 °C und/oder höchstens 120 °C, insbesondere bei etwa 100 °C.

Vorzugsweise erfolgt die Trocknung der zumindest einen wirkstoffhaltigen Matrix für eine Trocknungsdauer von zumindest 4 min und/oder von höchstens 16 min. Bevorzugt erfolgt die Trocknung bei der ersten Trocknungstemperatur für zumindest 1 min und/oder für höchstens 4 min, besonders bevorzugt für höchstens 3 min, insbesondere für 2 min.

Bevorzugt erfolgt die Trocknung bei der zweiten Trocknungstemperatur für zumindest 1 min und/oder für höchstens 4 min, besonders bevorzugt für höchstens 3 min, insbesondere für 2 min. Bevorzugt erfolgt die Trocknung bei der dritten Trocknungstemperatur für zumindest 0,5 min, und/oder für höchstens 4 min, besonders bevorzugt für höchstens 3 min, insbesondere für 1 min. Besonders bevorzugt erfolgt die Trocknung auch bei der vierten Trocknungstemperatur für zumindest 0,5 min und/oder für höchstens 4 min, besonders bevorzugt für höchstens 3 min, insbesondere für 1 min.

Vorteilhaft kann die Trocknung der wirkstoffhaltigen Matrix so erfolgen, dass ein bestimmter Restanteil an Lösungsmittel, vorzugsweise an Ethylacetat und/oder Heptan, insbesondere an Ethylacetat, in der getrockneten Matrix erreicht wird. Bevorzugt kann die wirkstoffhaltige Matrix so hergestellt werden, insbesondere so getrocknet werden, dass ein Gehalt an Ethylacetat und/oder Heptan, insbesondere an Ethylacetat, in der getrockneten Matrix in einem Bereich von zumindest etwa 100 ppm und/oder höchstens etwa 1500 ppm liegt. Bevorzugt kann die Matrix so getrocknet werden, dass ein Gehalt an Ethylacetat und/oder Heptan, insbesondere an Ethylacetat, in der getrockneten Matrix vorzugsweise nicht mehr als 500 ppm, besonders bevorzugt nicht mehr als 300 ppm, insbesondere nicht mehr 200 ppm, beträgt.

In einer bevorzugten Ausführung des Herstellungsverfahrens kann die wirkstoffhaltige Matrix, insbesondere das getrocknete wirkstoffhaltige Laminat, mit einer Folie, insbesondere einer Rückschicht wie beispielsweise einer Polyethylen Schutzfolie vom Typ CoTran 9720 des Herstellers 3M (USA), kaschiert werden.

Vorzugsweise kann die getrocknete wirkstoffhaltige Matrix in einem weiteren Schritt gestanzt werden, um flächige Wirkstoffkerne zu erhalten. Bevorzugt kann das Stanzen unmittelbar nach der Trocknung der wirkstoffhaltigen Matrix erfolgen. Die erhaltenen Wirkstoffkerne bzw. TDS können eine Größe von zumindest etwa 10 cm², bevorzugt von zumindest etwa 20 cm² und/oder von höchstens etwa 60 cm², bevorzugt von höchstens etwa 50 cm², insbesondere von etwa 30 bis 40 cm², haben.

Bevorzugt werden die so erhaltenen Wirkstoffkerne bzw. TDS möglichst zeitnah nach der Vereinzelung primärverpackt. Als Verpackungsmaterial eignet sich z. B. Papier/Aluminium/Surlyn-Verbundfolie des Herstellers Fa. JG Service AG, Deutschland oder auch PET/Aluminium/PE-Verbundfolie des Herstellers Huhtamaki, Deutschland.

Besonders bevorzugt erfolgt das Verpacken so, dass die jeweiligen TDS im Wesentlichen luftdicht verpackt sind. Luftdichtes Verpacken bedeutet hier, dass die TDS vorzugsweise unter Ausschluss von Sauerstoff verpackt sind. Bevorzugt kann in der Verpackung eine zur stabilen Lagerung der TDS vorteilhafte Atmosphäre ausgebildet werden, z. B. indem ein Innenraum der Verpackung mit einem Schutzgas, z. B. Stickstoff, begast wird.

Im Anschluss können die so verpackten TDS in einem optionalen Schritt temperiert werden. Die Temperierung erfolgt bei einer Temperatur von zumindest 7 °C, vorzugsweise von zumindest 8 °C, bevorzugt von zumindest 9 °C, besonders bevorzugt von zumindest 10 °C, insbesondere bevorzugt von zumindest 11 °C, oberhalb eines Schmelzpunkts des Aromatasehemmers der wirkstoffhaltigen Matrix, insbesondere oberhalb des Schmelzpunkts bzw. Schmelzbereichs von Anastrozol. Weiterhin erfolgt das Temperieren bei einer Temperatur von höchstens 20 °C, vorzugsweise von höchstens 15 °C, bevorzugt von höchstens 14 °C, besonders bevorzugt von höchstens 13 °C, insbesondere bevorzugt von 12 °C, oberhalb der Schmelzpunkt des Aromatasehemmers, insbesondere oberhalb des Schmelzpunkts bzw. Schmelzbereichs von Anastrozol.

Die Temperierung der verpackten TDS erfolgt für eine Dauer im Bereich von zumindest 1 Minute und/oder höchstens 5 Stunden. Vorzugsweise erfolgt die Temperierung für eine Zeitspanne von zumindest 10 Minuten, bevorzugt von zumindest 20 Minuten, besonders bevorzugt von zumindest 30 Minuten. Weiterhin erfolgt die Temperierung vorzugsweise für eine Dauer von höchstens 60 Minuten, bevorzugt von höchstens 50 Minuten, besonders bevorzugt von höchstens 40 Minuten, insbesondere von höchstens 35 Minuten, insbesondere bevorzugt von 30 Minuten.

Vorteilhafterweise können die TDS so hergestellt werden, dass der Wirkstoff aus der Klasse der Aromatasehemmer auch nach einer Lagerungszeit von mindestens 6 Monaten bis zu mehreren Jahren kristallfrei in der zumindest einen Matrix vorliegt, wobei eine gleichbleibende, kontinuierliche und kontrollierte Wirkstoffabgabe aus dem TDS möglich ist.

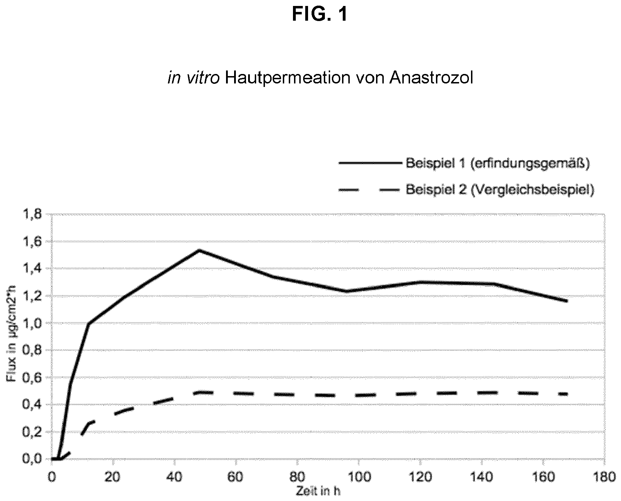

Weitere Merkmale der Erfindung ergeben sich aus der vorliegenden Beschreibung von Ausführungsbeispielen in Verbindung mit den Ansprüchen und den Figuren. Die einzelnen Merkmale können bei einer Ausführungform gemäß der Erfindung je für sich oder zu mehreren verwirklicht sein und schränken den Schutzumfang der vorliegenden Erfindung nicht ein. Es zeigen
- Fig. 1:: ein Diagramm einer *in vitro* Hautpermeation des Wirkstoffs Anastrozol aus einem erfindungsgemäßen TDS durch Humanhaut, im Vergleich zu einem nicht erfindungsgemäßen TDS.

### Beispiele

### Beispiel 1: erfindungsgemäßes Beispiel

Beispiel 1 zeigt im Folgenden die Herstellung erfindungsgemäßer transdermaler therapeutischer Systeme inklusive einer Stabilitätsstudie.

Es wird zunächst eine Mischung bereitgestellt, die 72 Gew.-% eines Polymersystems auf Basis von Silicon enthält. Bei dem Polymersystem handelt es sich um einen Amin-kompatiblen Silicon-Kleber (BioPSA 7-4301) des Herstellers Dow Corning (USA). Der Silicon-Kleber wurde zuvor mittels Ethylacetat (AppliChem, Deutschland) auf einen Feststoffgehalt von etwa 50 % eingestellt. Die bereitgestellte Mischung umfasst weiterhin 5 Gew.-% Lauryllactat (Schercemol LL Ester; Hersteller Lubrizol Advanced Materials) sowie 10 Gew.-% Dipropylenglykol (Dipropylenglykol Care; Hersteller BASF, Deutschland) und 5 Gew.-% Siliziumdioxid (Siliziumdioxid Ph. Eur., hochreine und amorphe hochdisperse Kieselsäure, hydrophil, Aerosil 200 Pharma; Hersteller Evonik, Deutschland). Als Wirkstoff umfasst die bereitgestellte Mischung 8 Gew.-% Anastrozol (Anastrozole; Hersteller Hetero Labs, Indien), welches in der Mischung überwiegend gelöst vorliegt. Der Feststoffanteil der bereitgestellten Lösung beträgt etwa 45 %.

Die so erhaltene Mischung wird auf einer einseitig fluoropolymerisierten Polyester Folie (PETP, 75 µm; Hersteller 3M; USA) ausgestrichen. Das Aufbringen der Mischung auf die Folie erfolgt so, dass nach dem Trocknen der Mischung eine Matrixschicht mit einem Flächengewicht von etwa 80 g/m² entsteht.

Die ausgestrichene Lösung wird gemäß dem nachfolgenden Trocknungsverfahren getrocknet: In einem ersten Schritt wird die Matrix bei einer Temperatur von 40 °C für eine Dauer von 1 Minute getrocknet. In einem zweiten (direkt anschließenden) Schritt erfolgt die Trocknung bei einer Temperatur von etwa 60 °C für 1 Minute. In einem dritten/vierten (direkt anschließenden) Schritt wird die Matrix bei etwa 100 °C für 2 Minuten getrocknet. In Folge der Trocknung wird das Ethylacetat bis auf einen Restanteil aus der Mischung bzw. der Matrixschicht entfernt: der Restanteil von Ethylacetat in der getrockneten Matrix lag bei < 2000 ppm. Die entstandene Matrixschicht wird mit einer Polyethylen-Schutzfolie (CoTran 9720; Hersteller 3M; USA) kaschiert.

Das so erhaltene Laminat wird unmittelbar nach der Trocknung auf eine Größe von ca. 40 cm² zugeschnitten, z. B. gestanzt, und einzeln primärverpackt. Die Verpackung erfolgt im Wesentlichen luftdicht. Im vorliegenden Fall ist das Verpackungsmaterial der TDS ein Aluminium-beschichteter Beutel aus Polyethylenterephthalat.

In einem weiteren (optionalen) Schritt, werden die verpackten TDS im Beutel für 30 Minuten bei einer Temperatur von 95 °C temperiert.

Die erfindungsgemäßen transdermalen therapeutischen Systeme wurden bei Temperaturen von 5°C und 25 °C/60 % r. F. (relative Luftfeuchtigkeit) gelagert. Die Stabilitätsstudien ergaben, dass sich weder unmittelbar nach der Herstellung noch innerhalb des Testzeitraums von mehr als 6 Monaten Wirkstoffkristalle gebildet hatten; ein Schmelzpunkt bzw. ein Schmelzbereich des Wirkstoffs konnte daher mittels DSC nicht nachgewiesen werden.

Eine Hautpermeation der hergestellten TDS wurde an Humanhaut an modifizierten Franz-Zellen über eine Dauer von 7 Tagen durchgeführt. Als Medium wurde HEPES-Puffer verwendet. Die erhaltenen Probelösungen wurden mittels HPLC analysiert.

Die Ergebnisse der *in vitro* Hautpermeation sind in Figur 1 dargestellt.

### Beispiel 2: Vergleichsbeispiel

Es wird zunächst eine Mischung bereitgestellt, die 84 Gew.-% eines Polymersystems auf Basis von Silicon enthält. Bei dem Polymersystem handelt es sich um einen Amin-kompatiblen Silicon-Kleber (BioPSA 7-4301) des Herstellers Dow Corning (USA). Der Silicon-Kleber wurde zuvor mittels Ethylacetat (AppliChem, Deutschland) auf einen Feststoffanteil von etwa 50 % eingestellt. Die bereitgestellte Mischung umfasst weiterhin 8 Gew.-% Glycerol (Glycerin, Hersteller AppliChem, Deutschland). Als Wirkstoff umfasst die bereitgestellte Mischung 8 Gew.-% Anastrozol (Anastrozole; Hersteller Hetero Labs, Indien), welcher überwiegend gelöst vorliegt. Der Feststoffanteil der bereitgestellten Mischung beträgt etwa 45 %.

Die so erhaltene Mischung wird auf einer einseitig fluoropolymerisierten Polyester Folie (PETP, 75 µm; Hersteller 3M; USA) ausgestrichen. Das Aufbringen der Mischung auf die Folie erfolgt so, dass nach dem Trocknen der Mischung eine Matrixschicht mit einem Flächengewicht von etwa 90 g/m² entsteht.

Die weitere Herstellung erfolgt analog zu Beispiel 1.

Die Ergebnisse der *in vitro* Hautpermeation von Anastrozol aus dem erfindungsgemäßen TDS (gemäß Beispiel 1) im Vergleich zu einem nicht erfindungsgemäßen TDS (Beispiel 2: Vergleichsbeispiel) sind in Figur 1 dargestellt.

Wie aus Figur 1 ersichtlich, zeigt die erfindungsgemäße Zusammensetzung im Vergleich zu einer nicht erfindungsgemäßen Zusammensetzung eine deutlich höhere Permeationsrate.

Die Hautpermeationsrate im Vergleich zu einem TDS, welches unmittelbar nach der Herstellung getestet wurde, ändert sich dabei vorteilhaft auch nach Lagerung über einen Zeitraum von mehr als 6 Monate nicht (Daten nicht dargestellt).

Es wird abschließend noch einmal darauf hingewiesen, dass es sich bei den vorhergehend detailliert beschriebenen Beispielen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können.

## Patentansprüche

1. Transdermales Darreichungssystem umfassend
(i) zumindest eine wirkstoffhaltige Matrix,
(ii) eine bevorzugt wirkstoffundurchlässige Rückschicht, und
(iii) eine optionale applikationsseitige Schutzfolie,
wobei die wirkstoffhaltige Matrix zumindest einen Wirkstoff aus der Klasse der Aromatasehemmer umfasst,
wobei der Wirkstoff aus der Klasse der Aromatasehemmer im Wesentlichen gelöst vorliegt, und
wobei die wirkstoffhaltige Matrix zumindest ein Polymer aus der Klasse der Silicone, insbesondere aus der Klasse der Amin-kompatiblen Silicone, umfasst.

2. Transdermales Darreichungssystem gemäß Anspruch 1, **dadurch gekennzeichnet, dass** der Aromatasehemmer aus Anastrozol ausgewählt ist.

3. Transdermales Darreichungssystem gemäß einem der Ansprüche 1 oder 2, wobei das Polymer ein Lösemittel, welches ausgewählt ist aus Ethylacetat und/oder Heptan, insbesondere aus Ethylacetat, umfasst.

4. Transdermales Darreichungssystem gemäß einem der vorhergehenden Ansprüche, wobei die wirkstoffhaltige Matrix einen Hilfsstoff, welcher ausgewählt ist aus der Gruppe der Milchsäureester, insbesondere aus der Gruppe der Formel 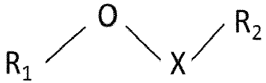 umfasst,
wobei R₁ -(CₙH₂ₙ₊₁) ist, wobei n= 11 bis 13, insbesondere n= 12, ist
wobei X ein Imin oder ein Carbonyl, insbesondere ein Carbonyl, ist und
wobei R₂ Methyl, Ethyl, -(CH(OH)-CH₃), oder -(CH₂CH₂OH), oder -(CH₂OH), insbesondere -(CH(OH)-CH3), ist.

5. Transdermales Darreichungssystem gemäß einem der vorhergehenden Ansprüche, wobei die wirkstoffhaltige Matrix einen Gehalt an Dipropylenglykol, insbesondere einen Gehalt von wenigstens etwa 0,5 Gew.-% und/oder höchstens 5 Gew.-%, umfasst.

6. Transdermales Darreichungssystem gemäß einem der vorhergehenden Ansprüche, wobei die wirkstoffhaltige Matrix einen Gehalt an Siliziumdioxid, insbesondere einen Gehalt von wenigstens etwa 1 Gew.-% und/oder höchstens etwa 10 Gew.-%, umfasst.

7. Verfahren zur Herstellung eines transdermalen Darreichungssystems, umfassend die Schritte:
(i) Bereitstellen zumindest einer Matrix enthaltend einen Wirkstoff aus der Klasse der Aromatasehemmer und ein Polymer aus der Klasse der Silicone,
(ii) Aufbringen der zumindest einen Matrix auf eine Folie zum Erhalt eines Laminats,
(iii) Trocknen des die zumindest eine Matrix umfassenden Laminats,
(iv) Kaschieren des die zumindest eine Matrix umfassenden Laminats aus Schritt (iii).

8. Verfahren zur Herstellung eines transdermalen Darreichungssystems gemäß Anspruch 7, wobei das Verfahren ferner umfasst:
(v) Stanzen von transdermalen Darreichungssystemen zum Erhalt von flächigen Wirkstoffkernen,
(vi) Verpacken, vorzugsweise luftdichtes Verpacken, von transdermalen Darreichungssystemen,
(vii) optional Temperieren der transdermalen Darreichungssysteme.

9. Verfahren zur Herstellung eines transdermalen Darreichungssystems gemäß Anspruch 8, wobei die Temperierung zumindest 7 °C, vorzugsweise zumindest 8 °C, bevorzugt zumindest 9 °C, besonders bevorzugt zumindest 10 °C, insbesondere bevorzugt zumindest 11 °C, und/oder höchstens 20 °C, vorzugsweise höchstens 15 °C, bevorzugt höchstens 14 °C, besonders bevorzugt höchstens 13 °C, insbesondere bevorzugt 12 °C, oberhalb eines Schmelzpunkts bzw. Schmelzbereichs des Aromatasehemmers erfolgt.

10. Verfahren zur Herstellung eines transdermalen Darreichungssystems gemäß Anspruch 8 oder 9, wobei die Temperierung für eine Dauer im Bereich von zumindest 1 min und/oder von höchstens 5 h erfolgt.

11. Transdermales Darreichungssystem gemäß einem der Ansprüche 1-6, wobei das transdermale Darreichungssystem nach einem Verfahren gemäß einem der Ansprüche 7 bis 10 erhältlich ist.

12. Transdermales Darreichungssystem gemäß einem der vorhergehenden Ansprüche zur medizinischen, tiermedizinischen oder kosmetischen Anwendung.

13. Transdermales Darreichungssystem gemäß einem der vorhergehenden Ansprüche zur Anwendung bei der Behandlung von Krebs, bevorzugt zur Behandlung eines Mammakarzinoms, insbesondere zur adjuvanten Behandlung eines hormonabhängigen Mammakarzinoms.

14. Transdermales Darreichungssystem gemäß einem der vorhergehenden Ansprüche, wobei das Transdermale Darreichungssystem für eine Applikationsdauer von mehr als 3 Tagen, vorzugsweise von mehr als 4 Tagen, bevorzugt von mehr als 5 Tagen, besonders bevorzugt von mehr als 6 Tagen, insbesondere bevorzugt von 7 Tagen, verwendet wird.
